# EUROPEAN PATENT SPECIFICATION

(11) **EP 1 477 495 B1**
(45) Date of publication and mention of the grant of the patent: **04.04.2007**
(21) Application number: 04000302.2
(22) Date of filing: 08.01.2004
(51) Int. Cl.: C07K 14/15

(54) **Antigenic fragments of the gp21 envelope protein of human T-Lymphotropic virus HTLV**
Antigene Fragmente vom gp21 Hüllprotein des humanen T-lymphotropen Virus HTLV
Fragments antigéniqes de la protéine gp21 du virus lymphotropique humain HTLV

(30) Priority: 25.04.2003 US 423156
(43) Date of publication of application: 17.11.2004
(73) Proprietor: DEVELOPMENT CENTER FOR BIOTECHNOLOGY, Taipei (TW)
(72) Inventor: Lin, Hsin-Yu, Banchiau City Taipei Taiwan 220 (TW); Hwong, Ching-Long, Tzuoying Chiu Kaohsiung Taiwan 813 (TW)
(74) Representative: TER MEER - STEINMEISTER & PARTNER GbR

(56) References cited:
- EP-A- 0 424 748
- WO-A-96/39630
- US-A- 5 643 174
- US-B1- 6 406 841
- GRAY G S ET AL: "ENVELOPE GENE SEQUENCE OF HTLV-1 ISOLATE MT-2 AND ITS COMPARISON WITH OTHER HTLV-1 ISOLATES" NORDISK CELLULOSA, STOCKHOLM, SE, vol. 177, 1990, pages 391-395, XP008014557 ISSN: 0281-6733
- YAMANO Y ET AL: "Preferential recognition of synthetic peptides from HTLV-I gp21 envelope protein by HLA-DRB1 alleles associated with HAM/TSP (HTLV-I-associated myelopathy/tropical spastic paraparesis)." JOURNAL OF NEUROIMMUNOLOGY. JUN 1997, vol. 76, no. 1-2, June 1997 (1997-06), pages 50-60, XP002295537 ISSN: 0165-5728
- HERNÁNDEZ MARIN M ET AL: "Chimeric synthetic peptides from the envelope (gp46) and the transmembrane (gp21) glycoproteins for the detection of antibodies to human T-cell leukemia virus type II." BIOCHEMICAL AND BIOPHYSICAL RESEARCH COMMUNICATIONS. 23 NOV 2001, vol. 289, no. 1, 23 November 2001 (2001-11-23), pages 7-12, XP002295538 ISSN: 0006-291X
- MADHU VARMA ET AL: "ENHANCED SPECIFICITY OF TRUNCATED TRANSMEMBRANE PROTEIN FOR SEROLOGIC CONFIRMATION OF HUMAN T-CELL LYMPHOTROPIC VIRUS TYPE 1 (HTLV-1) AND HTLV-2 INFECTIONS BY WESTERN BLOT (IMMUNOBLOT) ASSAY CONTAINING RECOMBINANT ENVELOPE GLYCOPROTEINS" JOURNAL OF CLINICAL MICROBIOLOGY, WASHINGTON, DC, US, vol. 33, no. 12, 1 December 1995 (1995-12-01), pages 3239-3244, XP000607560 ISSN: 0095-1137
- TALLET B ET AL: "One-step chromatographic purification procedure of a His-tag recombinant carboxyl half part of the HTLV-I surface envelope glycoprotein overexpressed in Escherichia coli as a secreted form" JOURNAL OF CHROMATOGRAPHY. BIOMEDICAL APPLICATIONS, ELSEVIER, AMSTERDAM, NL, vol. 753, no. 1, 25 March 2001 (2001-03-25), pages 17-22, XP004230259 ISSN: 0378-4347
- WANG BIN ET AL: "Molecular cloning, expression, and biological characterization of an HTLV-II envelope glycoprotein: HIV-1 expression is permissive for HTLV-II-induced cell fusion" AIDS RESEARCH AND HUMAN RETROVIRUSES, NEW YORK, NY, US, vol. 9, no. 9, September 1993 (1993-09), pages 849-860, XP000925933 ISSN: 0889-2229
- HORAL P: "IDENTIFICATION OF TYPE-SPECIFIC LINEAR EPITOPES IN THE GLYCOPROTEINS GP46 AND GP21 OF HUMAN T-CELL LEUKEMIA VIRUSES TYPE I AND TYPE II USING SYNTHETIC PEPTIDES" PROCEEDINGS OF THE NATIONAL ACADEMY OF SCIENCES OF USA, NATIONAL ACADEMY OF SCIENCE. WASHINGTON, US, vol. 88, no. 13, 1 July 1991 (1991-07-01), pages 5754-5758, XP000215690 ISSN: 0027-8424
- PALKER T J ET AL: "MAPPING OF IMMUNOGENIC REGIONS OF HUMAN T CELL LEUKEMIA VIRUS TYPE I (HTLV-I) GP46 AND GP21 ENVELOPE GLYCOPROTEINS WITH ENV-ENCODED SYNTHETIC PEPTIDES AND A MONOCLONAL ANTIBODY TO GP461" JOURNAL OF IMMUNOLOGY, THE WILLIAMS AND WILKINS CO. BALTIMORE, US, vol. 142, no. 3, 1 February 1989 (1989-02-01), pages 971-978, XP000268575 ISSN: 0022-1767
- KITZE B ET AL: "Human CD4+ T lymphocytes recognize a highly conserved epitope of human T lymphotropic virus type 1 (HTLV-1) env gp21 restricted by HLA DRB1*0101." CLINICAL AND EXPERIMENTAL IMMUNOLOGY. FEB 1998, vol. 111, no. 2, February 1998 (1998-02), pages 278-285, XP002295539 ISSN: 0009-9104
- DATABASE UNIPROT [Online] 1 October 1999 (1999-10-01), XP002295541 Database accession no. Q9WJZ9
- DATABASE UNIPROT [Online] 1 November 1996 (1996-11-01), XP002295542 Database accession no. Q82316
- DATABASE UNIPROT [Online] 1 November 1996 (1996-11-01), XP002295543 Database accession no. Q82317
- DATABASE UNIPROT [Online] 1 November 1996 (1996-11-01), XP002295544 Database accession no. Q82315
- DATABASE UNIPROT [Online] 1 October 2000 (2000-10-01), XP002295545 Database accession no. Q9JFQ4
- DATABASE UNIPROT [Online] 1 November 1996 (1996-11-01), XP002295546 Database accession no. Q80806
- HERNÁNDEZ MARIN M ET AL: "Chimeric synthetic peptides containing two immunodominant epitopes from the envelope gp46 and the transmembrane gp21 glycoproteins of HTLV-I virus." BIOCHEMICAL AND BIOPHYSICAL RESEARCH COMMUNICATIONS. 23 NOV 2001, vol. 289, no. 1, 23 November 2001 (2001-11-23), pages 1-6, XP002295540 ISSN: 0006-291X
- SHIMOTOHNO K ET AL: "COMPLETE NUCLEOTIDE SEQUENCE OF AN INFECTIOUS CLONE OF HUMAN T-CELLLEUKEMIA VIRUS TYPE II: AN OPEN READING FRAME FOR THE PROTEASE GENE" PROCEEDINGS OF THE NATIONAL ACADEMY OF SCIENCES OF USA, NATIONAL ACADEMY OF SCIENCE. WASHINGTON, US, vol. 82, no. 10, 1 May 1985 (1985-05-01), pages 3101-3105, XP000578026 ISSN: 0027-8424

## Description

### BACKGROUND OF THE INVENTION

### Field of the Invention

The present invention relates to human T-lymphotropic virus (HTLV). More particularly, the present invention relates to antigenic fragments of HTLV.

### Description of the Related Arts

Human T-lymphotropic virus (HTLV) classified into Retroviridae was the first human retrovirus to be isolated. HTLV type I was first isolated in 1978 whereas HTLV type II was in 1982. HTLV is spread by sexual contact, from mother to child and through contaminated blood product. It is endemic in southern Japan, Caribbean, South Africa and Melanesia. To avoid viral transmission, screening of blood donations for HTLV is now routinely carried out in many countries. Since 1996, antibodies of HTLV-I and HTLV-II have been screening by ELISA and western blotting in Taiwan.

The preliminary screening of HTLV is carried out by ELISA, and a final diagnosis can be made by western blotting and polymerase chain reaction. The commercialized HTLV assay utilizes viral total lysate as antigen to detect specific antibodies from carrier blood. For higher sensitivity and specificity, a peptide fragment of viral envelop can be used as an additional antigen. The preparation of viral total lysate is complicated and has a potential risk; there is, therefore, still a need for a safe and effective HTLV antigen.

### SUMMARY OF THE INVENTION

It is therefore a primary object of the present invention to provide fusion proteins of human T-lmphotropic virus (HTLV) with Glutathione S-transferase (GST) or Thioredoxin. The fusion proteins have the advantage of high specific and sensitive to HTLV-I/II and the preparation of these proteins is safe and effective. In addition, the fusion proteins can be applied in HTLV-I/II assay. Using genomic engineering, the antigenic viral recombinant protein expressed by E. coli can be prepared in large quantities at low cost. Moreover, avoiding the cultivation and purification of HTLV, the preparation of the present invention is safer than the current preparation.

Accordingly, in a reference aspect, the invention features an isolated peptide comprising an antigenic fragment of HTLV-I gp21 having the amino acid sequence of SEQ ID No: 3.

The reference aspect also features an isolated peptide comprising an antigenic fragment of HTLV-II gp21 having the amino acid sequence of SEQ ID No: 4.

In addition, the reference aspect features an isolated nucleic acid encoding an antigenic fragment of HTLV-I gp21, wherein the nucleic acid comprises the nucleotide sequence of SEQ ID No: 55.

The reference aspect also features an isolated nucleic acid encoding an antigenic fragment of HTLV-II gp21, wherein the nucleic acid comprises the nucleotide sequence of SEQ ID No: 56.

In the invention, both of the aforementioned nucleic acids encoding antigenic fragments in the invention are ² combined with glutathione S-transferase(GST) or thioredoxin(thio) to form 4 recombinant nucleic acids as below.
1. A nucleic acid as specified in claim 5 encoding GST/HTLV-I gp21 fusion protein, wherein the nucleic acid consists of the nucleotide sequence of SEQ ID No: 57.
2. A nucleic acid as specified in claim 6 encoding Thio/HTLV-I gp21 fusion protein, wherein the nucleic acid consists of the nucleotide sequence of SEQ ID No: 59.
3. A nucleic acid as specified in claim 7 encoding GST/HTLV-II gp21 fusion protein, wherein the nucleic acid consists of the nucleotide sequence of SEQ ID No: 58.
4. A nucleic acid as specified in claim 8 encoding Thio/HTLV-II gp21 fusion protein, wherein the nucleic acid consists of the nucleotide sequence of SEQ ID No: 60.

In addition, the present invention also features an expression vector comprising a nucleic acid encoding any of the four fusion proteins operably linked to a nucleotide sequence regulatory element that controls expression of the nuleic acid and a process for producing the HTLV antigenic fragments. The process comprises introducing an expression vector comprising a nucleic acid encoding any of the four fusion proteins into a cell, culturing the cell under conditions suitable for production of the fusion protein, and recovering the fusion protein from the cell culture.

In one embodiment of the process, the cell is *Escherichia coli,* for example, BL21(DE3) strain. For the production of GST/HTLV gp21 fusion protein, recovery is enabled by glutathione sepharose column; for the production of Thio/HTLV gp21 fusion protein, recovery is enabled by Ni-NTA column.

Accordingly, the four nucleic acids encode four fusion proteins as below.
1. GST/HTLV-I gp21 fusion protein as claimed in claim 1 consisting of the amino acid sequence of SEQ ID No: 5.
2. Thio/HTLV-I gp21 fusion protein as claimed in claim 2 consisting of the amino acid sequence of SEQ ID No: 7.
3. GST/HTLV-II gp21 fusion protein as claimed in claim 3 consisting of the amino acid sequence of SEQ ID No: 6.
4. Thio/HTLV-II gp21 fusion protein as claimed in claim 4 consisting of the amino acid sequence of SEQ ID No: 8.

Another aspect of the invention features a kit for the detection of human T-lymphotrophic virus (HTLV). The kit comprises a solid substrate, a first HTLV gp21 antigenic fragment immobilized on the solid substrate, a blocking solution for blocking a HTLV gp21 antigenic fragment-unbound region on the solid substrate, a second HTLV gp21 antigenic fragment, a wash solution, and a signal-producing means operably linked to the second HTLV gp21 antigenic fragment to produce a signal, wherein the first and second HTLV gp21 are selected from any of the fusion proteins.

In one embodiment of the kit in the invention, the first HTLV gp21 antigenic fragment is Thio/HTLV-II gp21 fusion protein as specified in claim 4, and the second HTLV gp21 antigenic fragment is GST/HTL-I gp21 fusion protein as specified in claim 1.

### BRIEF DESCRIPTION OF THE DRAWINGS

The present invention will be more fully understood and further advantages will become apparent when reference is made to the following description of the invention and the accompanying drawings in which:
FIGS. 1A and 1B show nucleotide sequences of HTLV antigenic fragment. FIG. 1A represents the entire sequence of HTLV-I gp21; and FIG. 1B represents the entire sequence of HTLV-II gp21.
FIG. 2A-2C shows vector pGEX-KG (2A), and the construct pGST/HTVL-I gp21 (2B) as well as the construct pGST/HTLV-II gp21 (2C).
FIG. 3A-3C shows vector pThioHis B (3A), and the construct pThio/HTLV-I gp21 (3B) as well as the construct pThio/HTLV-II gp21 (3C).
FIG. 4 represents a SDS-PAGE analysis for purified GST/HTLV-I gp21 (lane 1) and GST/HTLV-II gp21 (lane 2) fusion proteins (33 kDa).
FIG. 5 represents a SDS-PAGE analysis for purified Thio/HTLV-I gp21 (lane 1) and Thio/HTLV-II gp21 (lane 2) fusion proteins (25 kDa).

### DETAILED DESCRIPTION OF THE INVENTION

HTLV is classified as HTLV-I and HTLV-II; diseases caused by HTLV-I include adult T-cell leukemia/lymphoma (ATL) developed by about 2-3% of infected patients, HTLV-I-associated myelopathy/tropical spastic paraparesis (HAM/TSP) developed by only 2-3% of infected patients, generally appearing in middle age(40-50 yrs). For those infected by transfusion, however, the duration shortens to one month to 4 years, with 95% of infected patients experiencing no lifelong symptoms. HTLV-II is considered to act as associated with atypical hairy cell leukemia.

HTLV genome is ss(+)RNA composed of *gag*, *pol, env, tax* and *rex* genes. *gag* gene is translated into a polyprotein, and then spliced into mature core proteins: p19, p24, p15, etc. *pol* gene is translated into reverse transcriptase, integrase, and RNAse H. *env* gene is translated into envelop proteins p21 and p46. *tax* and *rex* genes are associated with viral replication. After infection, HTLV induces human antibodies against viral *gag* protein, mainly p24. The antibody arises 2 months after infection and then antibodies against viral surface protein are produced.

The present invention is based on the discovery of a region rich in antigenic determinants in HTLV-I gp21 (SEQ ID No: 1) and HTLV-II gp21 (SEQ ID No:2) by antigenic determinant analysis of HTLV-I/II. The region is shown below.

The frame regions indicate antigenic determinants.

Using assembly PCR, modified HTLV-I gp21 and HTLV-II gp21 genes were synthesized according to the gene sequence from genebank. The HTLV-I gp21 gene was modified with E. coli preferred codons. Using these sequences as templates, gp21 fragments with 270 bp were amplified by PCR and cloned into pGEX-KG or pThioHisB.

Therefore, the reference aspect of the present invention features two isolated peptides, an isolated peptide comprising an antigenic fragment of HTLV-1 gp21 having the amino acid sequence of SEQ ID No: 3, and an isolated peptide comprising an antigenic fragment of HTLV-II gp21 having the amino acid sequence of SEQ ID No: 4.

The two peptides of the reference aspect of the present invention are encoded from two nucleic acids, an isolated nucleic acid encoding an antigenic fragment of HTLV-I gp21, wherein the nucleic acid comprises the nucleotide sequence of SEQ ID No: 55, and an isolated nucleic acid encoding an antigenic fragment of HTLV-II gp21, wherein the nucleic acid comprises the nucleotide sequence of SEQ ID No: 56.

In the invention any of the aforementioned antigenic fragments is combined with glutathione S-transferase (GST) or thioredoxin(thio) to form 4 recombinant nucleic acids as below.
1. A nucleic acid as specified in claim 5 encoding GST/HTLV-I gp21 fusion protein, wherein the nucleic acid consists of the nucleotide sequence of SEQ ID No: 57.
2. A nucleic acid as specified in claim 6 encoding Thio/HTLV-1 gp21 fusion protein, wherein the nucleic acid consists of the nucleotide sequence of SEQ ID No: 59.
3. A nucleic acid as specified in claim 7 encoding GST/HTLV-II gp21 fusion protein, wherein the nucleic acid consists of the nucleotide sequence of SEQ ID No: 58.
4. A nucleic acid as specified in claim 8 encoding Thio/HTLV-II gp21 fusion protein, wherein the nucleic acid consists of the nucleotide sequence of SEQ ID No: 60.

In addition, the scope of the invention also includes an expression vector comprising a nucleic acid encoding any of the four fusion proteins operably linked to a nucleotide sequence regulatory element that controls expression of the nucleic acid, and a process for producing the HTLV antigenic fragments. The process comprises introducing a expression vector comprising a nucleic acid encoding any of the four fusion proteins into a cell, culturing the cell under conditions suitable for production of the fusion protein, and recovering the fusion protein from the cell culture.

Examples of the expression vector include pGST/HTLV-I gp21, pThio/HTLV-I gp21, pGST/HTLV-II gp21, and pThio/HTLV-II gp21. The aforementioned expression vectors have been deposited in the Bioresources collection and research center in Taiwan, Republic of China, and the depository numbers are 940407, 940405, 940408, and 940406, respectively. The afore mentioned expression vectors also have been deposited in the American Type Culture Collection (ATCC), 10801 Univeristy Blvd., Manassas, VA 20110-2209, USA, and the depository numbers are PTA-5238 for pGST/HTLV-1 gp21, PTA-5239 for pGST/HTLV-II gp21, PTA-5240 for pThio/HTLV-I gp21, and PTA-5241 for pThio/HTLV-II gp21, respectively.

In one embodiment of the process, the cell is Escherichia coli, for example, BL21 (DE3) strain. For the production of GST/HTLV gp21 fusion protein, recovery is enabled by glutathione sepharose column; for the production of Thio/HTLV gp21 fusion protein, recovery is enabled by Ni-NTA affinity column.

Accordingly, the four nucleic acids encode four fusion proteins as below.
1. GST/HTLV-I gp21 fusion protein as claimed in claim 1 consisting of the amino acid sequence of SEQ ID No: 5.
2. Thio/HTLV-I gp21 fusion protein as claimed in claim 2 consisting of the amino acid sequence of SEQ ID No: 7.
3. GST/HTLV-II gp21 fusion protein as claimed in claim 3 consisting of the amino acid sequence of SEQ ID No: 6.
4. Thio/HTLV-II gp21 fusion protein as claimed in claim 4 consisting of the amino acid sequence of SEQ ID No: 8.

The invention also features a kit for the detection of human T-lymphotrophic virus(HTLV). The kit comprises a solid substrate, a first HTLV gp21 antigenic fragment immobilized on the solid substrate, a blocking solution for blocking a HTLV gp21 antigenic fragment-unbound region on the solid substrate, a second HTLV gp21 antigenic fragment, a wash solution, and a signal-producing means operably linked to the second HTLV gp21 antigenic fragment to produce a signal, wherein the first and second HTLV gp21 are selected from any of the fusion proteins.

In one embodiment of the kit in the invention, the first HTLV gp21 antigenic fragment is Thio/HTLV-II gp21 fusion protein as specified in claim 4, and the second HTLV gp21 antigenic fragment is GST/HTLV-I gp21 fusion protein as specified in claim 1.

The solid substrate of the kit includes, but is not limited to, glass, silicon, ceramic, metal, or organic polymer such as styrene, ethylene, propylene, ester, acrylic acid, acrylic ester, alkyl acrylic acid, or alkyl acrylic ester.

The blocking solution includes a solution of BSA, casein, or gelatin.

The wash solution includes PBS, TBS, or PBST with 0.05% Tween 20.

The signal producing means includes radioactive label, fluorescent label, phosphorescent label, luminescent label, or enzyme. The luminescent label includes biological luminescent label or chemical luminescent label. The enzyme includes alkaline phosphatase, hydrogen peroxidase, or β-galactosidase. In one embodiment, the kit further comprises a substrate, and the susbtrate reacts with the enzyme to produce a color.

In another embodiment of the detection kit of HTLV, the signal producing means further includes a biotin and a avidin, the avidin operably binding to the radioactive label, fluorescent label, phosphorescent label, luminescent label, or enzyme. The enzyme includes alkaline phosphatase, hydrogen peroxidase, or β-galactosidase. In addition, the kit further comprises a substrate, and the susbtrate reacts with the enzyme to produce a color.

### Reference EXAMPLE 1 Assembly PCR for the synthesis of HTLV-I/II gp21

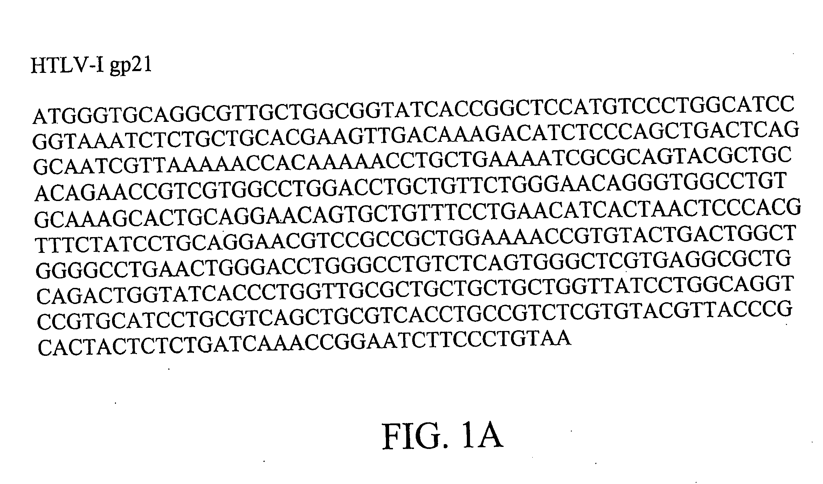

HTLV-I gp21 gene sequence from GeneBank D13784 was modified with E. coli preferred codons. 20 primers were designed for assembly PCR to synthesize HTLV-I gp21 of 505bp as shown in FIG. 1A (SEQ ID No: 9). The two ends were designed with *Nde* I (5') and *Xho* I (3') restriction sites. The 20 primers are shown below.

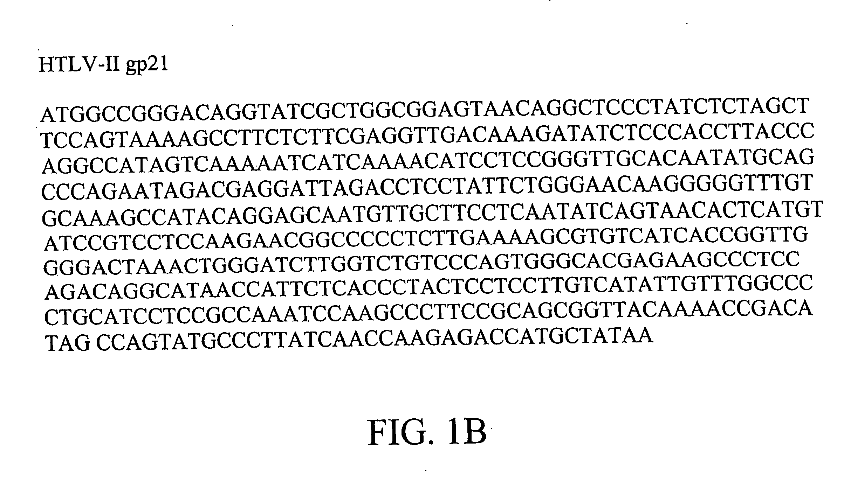

According to HTLV-II gp21 gene sequence from GeneBank NC_001488, 20 primers were designed for assembly PCR to synthesize HTLV-II gp21 of 514bp as shown in FIG. 1B (SEQ ID No: 30). The two ends were designed with *Nde* I (5') and *Xho* I (3') restriction sites. The 20 primers are shown below.

### Reference EXAMPLE 2 Construction of pHTLV gp21

The fragments amplified by assembly PCR were separated by 2% agarose gel and purified by QIAquick Gel Extraction Kit (QIAGEN). DNA was eluted with 50µl elution beffer (10 mM Tris-Cl, pH 8.5), and treated by *Nde* I and *Xho* I. pET15b was also treated by *Nde* I and *Xho* I, separated by 0.8% agarose gel, and purified by QIAquick Gel Extraction Kit (QIAGEN) to obtain a DNA fragment of 2900bp. Ligation of the pET15b and HTLV gp21 fragment was performed by DNA Ligation Kit (TaKaRa) at 16 °C for 40 min. The ligation product was transformed into DH5α competent cell. The recombinant constructs were analyzed and designated "pB119/HTLV-I gp21" and "pB119/HTLV-II gp21" respectively.

### EXAMPLE 3 Construction of pGST/HTLV-I/II gp21

Using pB119/HTLV-I gp21 or pB119/HTLV-II gp21 as template, HTLV gp21 antigenic fragment of 270 bp was amplified with two sets of primers: two ends of HTLV-I gp21 fragments were designed with *Nco*I (5') and *Hin*d III (3') restriction sites, and two ends of HTLV-II gp21 fragments were designed with *Bam*HI (5') and *Hin*d III(3') restriction sites.

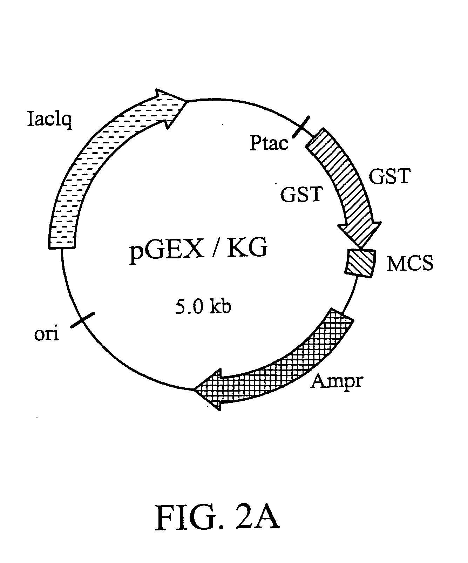

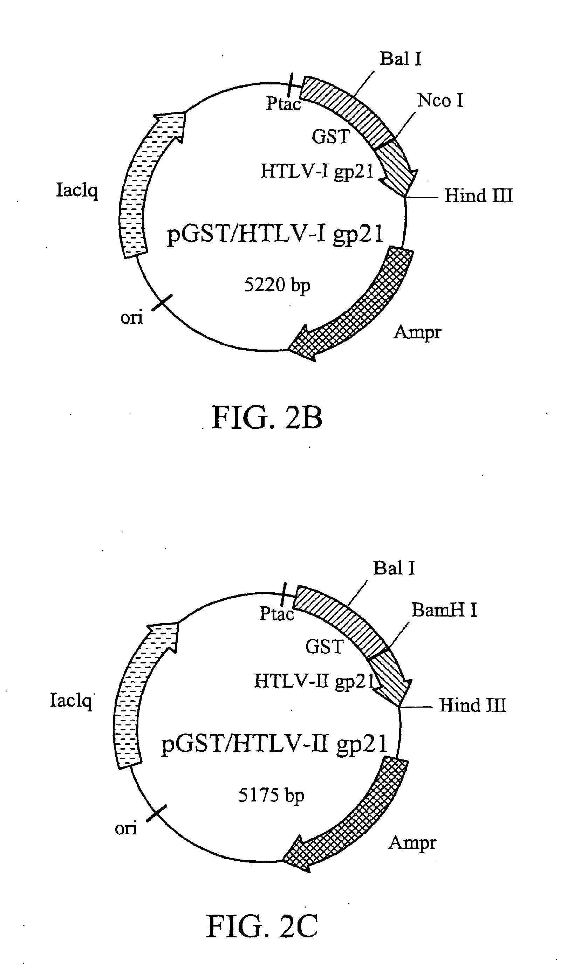

The fragments obtained are as shown in SEQ ID No: 55 and 56. The two fragments were cloned into pGEX-KG as in the map shown in FIG. 2A and designated pGST/HTLV-I gp21 and pGST/HTLV-II gp21, as shown in FIG. 2B and 2C, respectively. The nucleotide sequence of GST/HTLV-I gp21 is shown as SEQ ID No: 57, whereas that of GST/HTLV-II gp21 is shown as SEQ ID No: 58.

### EXAMPLE 4 Construction of pThioredoxin/HTLV-I/II gp21

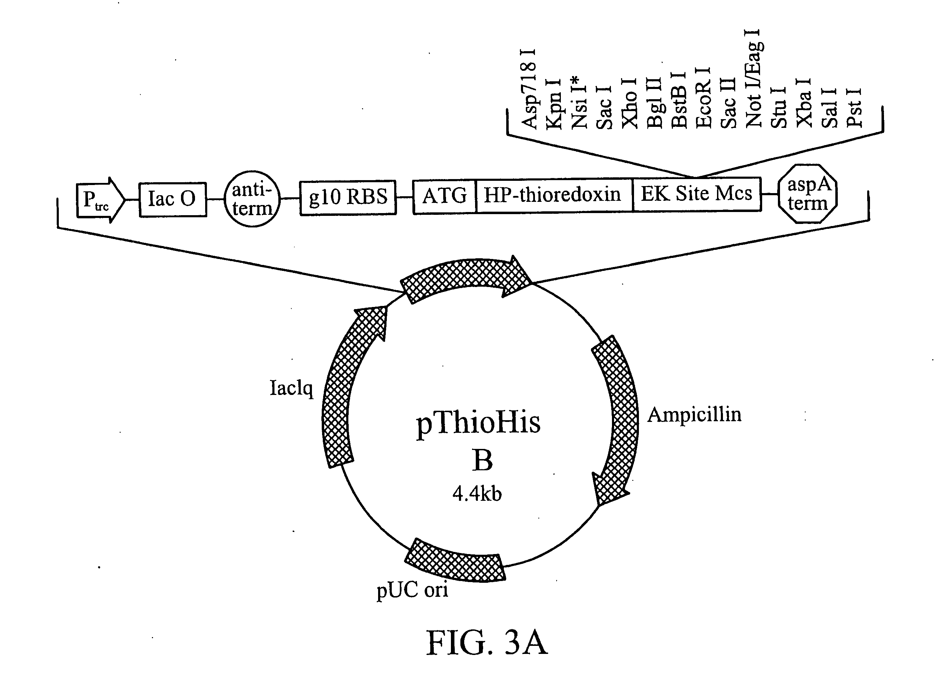

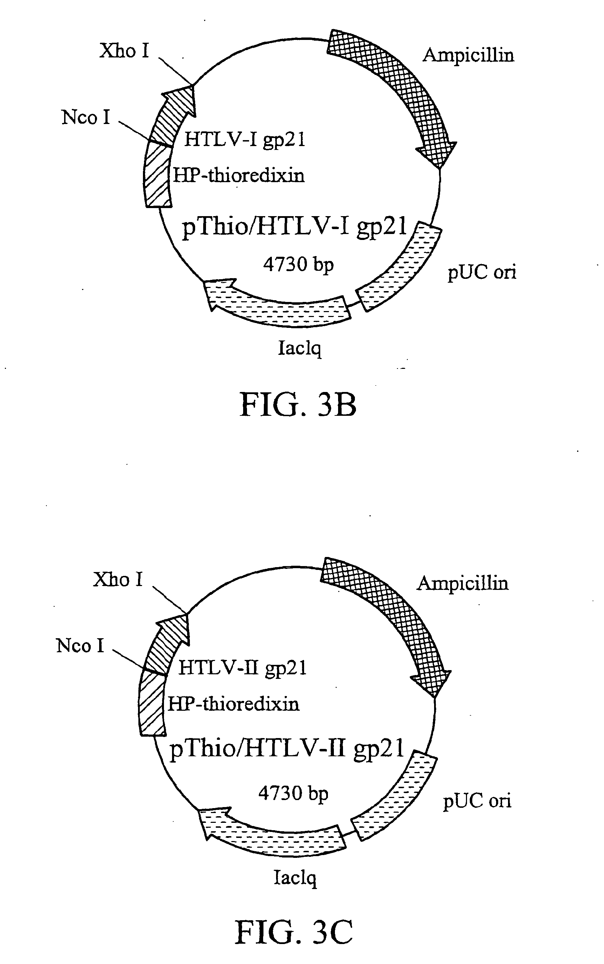

pB119/HTLV-I gp21 or pB119/HTLV-II gp21 were treated with *Nco* I (5') and *Xho* I(3') to obtain gp 21 fragments with 6 histidine on the N end. The gp21 fragments were subcloned into pThioHisB (Invitrogen) as the map shown in FIG. 3A. The resulting expression constructs were designated pThio/HTLV-I gp21 and pThio/HTLV-II gp21 as shown in FIG. 3B and 3C respectively. The nucleotide sequence of Thio/HTLV-I gp21 is shown as SEQ ID No: 59, whereas that of Thio/HTLV-II gp21 is as SEQ ID No: 60.

### EXAMPLE 5 IPTG induction of protein expression

The expression constructs were transformed into BL21(DE3) expression host and 200 rpm vibration-cultured in 1 L of LB/Amp at 37 °C . Bacteria were grown to O.D.₅₉₅=0.8, and 1 mL of the bacteria culture was sidelined for "expression control" (TO). 1 mL of 1M IPTG (isopropyl-b-D-thiogalactopyranoside) was added to the rest of the culture to induce protein expression. After 3hr induction, 1 mL of the bacteria culture was sidelined as Ta. The bacteria harvested at different time intervals were lysed with lysis buffer separately. The volume of the lysis buffer varies according to the equation of O.D.xvolume (µl)/20=lysis buffer volume (µl) An equal volume of sample buffer was then added and the reaction was heated at 95°C for 5 min. 10 µl of the sample was analyzed by SDS-PAGE. The rest of the culture was centrifuged at 8000rpm for 15 min to collect bacteria for subsequent preotein purification.

### EXAMPLE 6 Confirmation of the expressed protein forms

The centrifuged bacteria were resuspended and homogenized with 100 mL IMAC-5 with 0.1% Triton-100. The bacteria were lyzed by microfluidizer and centrifuged at 15000 rpm for 30 min to separate supernatant and pellet. The expressed protein was confirmed with SDS-PAGE for soluble form and inclusion body.

### EXAMPLE 7 Purification of GST fusion protein

GST/HTLV-I gp21 (33kDa) or GST/HTLV-II gp21 (33kDa) were purified by Glutathione Sepharose^{™} 4B (Amersham Pharmacia Biotech). The centrifuged bacteria were resuspended and homogenized with 100 mL IMAC-5 with 0.1% Triton-100. The bacteria were lyzed by microfluidizer and centrifuged at 15000 rpm for 30 min to separate supernatant. 2 mL Glutathione Sepharose^{™} 4B column was prepared and balanced with 10 mL IMAC-5 with 0.1% Triton-100. 50 mL supernatant was passed through the column twice. Unbound protein and impurities were washed out by 10 mL IMAC-5 with 0.1% Triton-100. Finally, GST fusion protein was eluted with 30 mL of 10mM Glutathione.

### EXAMPLE 8 Purification of Thioredoxin fusion protein by Ni-NTA affinity column

For the purification of Thio/HTLV-I gp21 and Thio/HTLV-II gp21 in the form of inclusion bodies, the unsoluble pellets after homogenization were resuspended with 60 mL IMAC-5 with 8M Urea and stirred oevernight. The solution was centrifuged at 15000 rpm for 30 min to obtain the supernatant. The supernatant was passed through 2.5mL Ni-NTA affinity column twice or 3 times. The impurities were washed away with the buffer listed below in sequence: Buffer B (8M urea, 0.1M NaH₂PO₄, 0.001M Tris. HCl, pH 8.0); Buffer C (8M urea, 0.1M NaH₂PO₄, 0.001M Tris.HCl, pH 6.3); Buffer D (8M urea, 0.1M NaH₂PO₄, 0.001M Tris.HCl, pH 5.9); Buffer E (8M urea, 0.1M NaH₂PO₄, 0.001M Tris.HCl, pH 4.5). The final protein was eluted with 8M urea containing 100mM EDTA. All steps were performed at room temperature.

### EXAMPLE 9 Analysis of HTLV-I/II antigenic protein

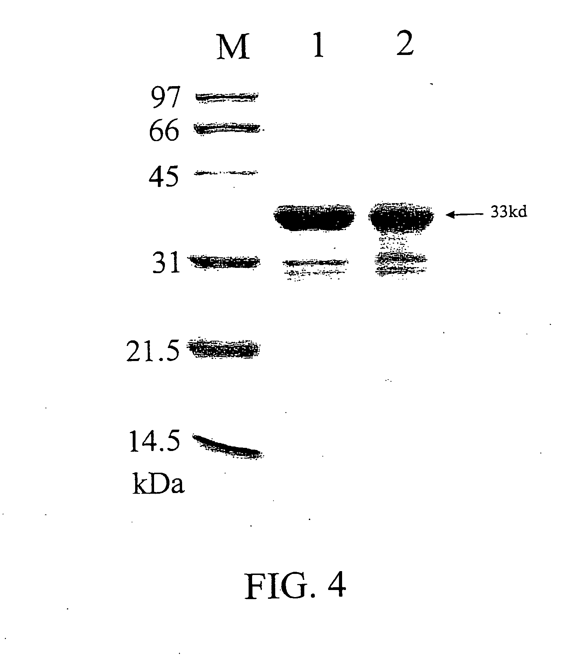

The fusion protein GST/HTLV-I/II gp21 was prepared to increase protein expression and soluble form protein. The results of purified fusion protein GST/HTLV-I/II gp21 are shown in FIG. 4, GST/HTLV-1 gp21 (lane 1) and GST/HTLV-II gp21 (lane 2) are both 33 kDa and purity over 90%.

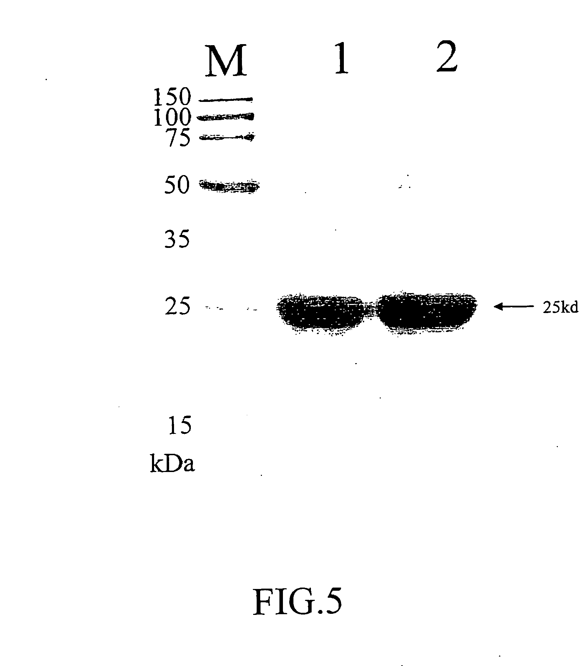

The fusion protein Thio/HTLV-I/II gp21 was prepared for direct sandwich ELISA which reduces non-specific signals in background. The results of purified fusion protein Thio/HTLV-I/II gp21 are shown in FIG. 5, Thio/HTLV-I gp21 (lane 1) and Thio/HTLV-II gp21 (lane 2) are both 25 kDa and purity over 95%.

### Example 10 Biotinylatin of antigenic protein

GST/HTLV-I/II gp21 (33 kDa) was concentrated to 1 mg/mL and dialyzed to PBS buffer. Dissolved biotin protein solution was added to the protein solution slowly with continuous mixing. After a 2-hour reaction at 4°C, Tris HCl was added to a final concentration of 50 mM to terminate the reaction. The biotin-labeld protein was then dialyzed to 50 mM Tris HCl for the subsequent direct sandwich ELISA.

### EXAMPLE 11 Assay of specificity and sensitivity for human sera

The preliminary test results show that Thio/HTLV-II gp21 can be used for coating in accompaniment with biotin-labeld GST/HTLV-I gp21. To investigate whether HTLV fusion protein has good sensitivity, HTLV sera standard control (Anti-HTLV I/II Mixed Titer Performance Panel, BBI) and sera from Tainan Blood Donation Center identified as positive by western blotting were used. Normal sera were used for testing specificity of the fusion protein. In the method described below, 1µg Thio/HTLV-II gp21 per well diluted in 100µl coating buffer (0.013M Na₂CO₃, 0.035M NaHCO₃, pH 9.6) was coated on 96-well plate. After 1 hour incubation at 37°C, the plate was washed with PBST (PBS with 0.05% Tween 20) three times. 200µl overcoating buffer (GBC corp.) was added per well to incubate at 37°C for 2 hours. The fluid was drawn out and the plate was stored at -20°C for the subsequent experiment. 100 µl 20× diluted sample sera in 5H Specimen Diluent C (GBC corp.) was added per well and the plate was incubated at 37°C for 1 hour. The plate was then washed with PBST six times. 100µl 250× diluted biotin-labeld GST/HTLV-I gp21 in 2Ha conjugate Diluent (GBC corp.) was added per well and the plate was incubated at 37°C for 1 hour. The plate was then washed with PBST six times. 100 µl Avidin conjugate AP (1:5000 dilution) in 2Ha conjugate Diluent (GBC corp.) was added per well and the plate was incubated at 37°C for 1 hour. The plate was then washed with PBST six times. 5 mg p-nitrophenyl phosphate (Sigma) was dissolved in 5 ml color developing buffer (10% Diethanolamin, 0.5 mM MgCl₂) as color developing solution. 100 µl color developing solution was added per well and the plate was incubated at 37°C for 15 min. The results were read at OD 405nm by ELISA reader (Bio-Rad Model 550).

The results show that the average absorbance is 1.052 for sera from Taiwan Blood Donation Center identified as positive by western blotting (19 samples of HTLV-I positive), 1.098 for Anti-HTLV I/II Mixed Titer Performance Panel (BBI)(7 samples of HTLV-I positive and 11 samples of HTLV-II positve), 0.1528 for 92 normal sera. The results have significant differences between positive and negative samples. The fusion proteins have good sensitivity and specificity, with both exceeding 99%. Therefore, the fusion protein can act as a detection agent for HTLV.

While the invention has been particularly shown and described with the reference to the preferred embodiments thereof, it will be understood by those skilled in the art that various changes in form and details may be made without departing from the spirit and scope of the invention.

## Claims

1. An isolated peptide consisting of a fusion protein of glutathione S-transferase (GST) and an antigenic fragment of HTLV-I gp21, with the amino acid sequence of SEQ ID No: 5.

2. An isolated peptide consisting of a fusion protein of thioredoxin and an antigenic fragment of HTLV-I gp21, with the amino acid sequence of SEQ ID No: 7.

3. An isolated peptide consisting of a fusion protein of glutathione S-transferase and an antigenic fragment of HTLV-II gp21, with the amino acid sequence of SEQ ID No: 6.

4. An isolated peptide consisting of a fusion protein of thioredoxin and an antigenic fragment of HTLV-II gp 21, with the amino acid sequence of SEQ ID No: 8.

5. An isolated nucleic acid, consisting of a nucleotide sequence of SEQ ID NO: 57 encoding the fusion protein GST/HTLV-I gp21 as specified in claim 1.

6. An isolated nucleic acid, consisting of a nucleotide sequence of SEQ ID NO: 59 encoding the fusion protein Thio/HTLV-I gp21 as specified in claim 2.

7. An isolated nucleic acid consisting of a nucleotide sequence of SEQ ID NO: 58 encoding the fusion protein GST/HTLV-II gp21 as specified in claim 3.

8. An isolated nucleic acid consisting of a nucleotide sequence of SEQ ID NO: 60 encoding the fusion protein Thio/HTLV-II gp21 as specified in claim 4.

9. An expression vector, comprising a nucleic acid encoding the fusion protein GST/HTLV-1 gp21 as specified in claim 1 operably linked to a nucleotide sequence regulatory element that controls expression of the nucleic acid, wherein the nucleic acid comprises the nucleotide sequence of SEQ ID NO: 57.

10. The expression vector as claimed in claim 9, wherein the expression vector is deposited in the American Type Culture Collection and assigned PTA-5238.

11. An expression vector, comprising a nucleic acid encoding the fusion protein Thio/HTLV-I gp21 as specified in claim 2 operably linked to a nucleotide sequence regulatory element that controls expression of the nucleic acid, wherein the nucleic acid comprises the nucleotide sequence of SEQ ID NO: 59.

12. The expression vector as claimed in claim 11, wherein the expression vector is deposited in the American Type Culture Collection and assigned PTA-5240.

13. An expression vector, comprising a nucleic acid encoding the fusion protein GST/HTLV-II gp21 as specified in claim 3 operably linked to a nucleotide sequence regulatory element that controls expression of the nucleic acid, wherein the nucleic acid comprises the nucleotide sequence of SEQ ID NO: 58.

14. The expression vector as claimed in claim 13, wherein the expression vector is deposited in the American type Culture Collection and assigned PTA-5239.

15. An expression vector, comprising a nucleic acid encoding the fusion protein Thio/HTLV-II gp21 as specified in claim 4 operably linked to a nucleotide sequence regulatory element that controls expression of the nucleic acid, wherein the nucleic acid comprises the nucleotide sequence of SEQ ID NO: 60.

16. The expression vector as claimed in claim 15, wherein the expression vector is deposited in the American Type Culture Colllection and assigned PTA-5241.

17. A process for producing an antigenic fragment of HTLV gp21, comprising:
introducing an expression vector as claimed in any one of claims 9-16 into a cell;
culturing the cell under conditions suitable for production of a protein; and
recovering the protein from the cell culture to obtain the antigenic fragment of HTLV gp21.

18. A kit for the detection of human T-lymphotropic virus (HTLV), comprising:
a solid substrate,
a first HTLV gp21 antigenic fragment immobilized on the solid substrate,
a blocking solution for blocking a HTLV gp21 antigenic fragment-unbound region on the solid substrate;
a second HTLV gp21 antigenic fragment,
a wash solution, and
a signal-producing means operably linked to the second HTLV gp21 antigenic fragment to produce a signal, wherein the first and the second HTLV gp21 antigenic fragments are selected from peptides as claimed in claims 1-4.

19. The kit as claimed in claim 18, wherein the first HTLV gp21 antigenic fragment is fusion protein Thio/HTLV-II gp21 as specified in claim 4 and the second HTLV gp21 antigenic fragment is fusion protein GST/HTLV-1 gp21 as specified in claim 1.

## Patentansprüche

1. Isoliertes Peptid, bestehend aus einem Fusionsprotein aus Glutathion-S-Transferase (GST) und einem antigenen Fragment von HTLV-I gp21 mit der Aminosäuresequenz von SEQ ID Nr: 5.

2. Isoliertes Peptid, bestehend aus einem Fusionsprotein von Thioredoxin und einem antigenen Fragment von HTLV-1 gp21 mit der Aminosäuresequenz von SEQ ID Nr: 7.

3. Isoliertes Peptid, bestehend aus einem Fusionsprotein von Glutathion-S-Transferase und einem antigenen Fragment von HTLV-II gp21 mit der Aminosäuresequenz von SEQ ID Nr: 6.

4. Isoliertes Peptid, bestehend aus einem Fusionsprotein aus Thioredoxin und einem antigenen Fragment von HTLV-II gp21 mit der Aminosäuresequenz von SEQ ID Nr: 8.

5. Isolierte Nucleinsäure, bestehend aus einer Nucleotid-Sequenz von SEQ ID Nr: 57, welche für das Fusionsprotein GST/HTLV-1 gp21, wie in Anspruch 1 spezifiziert, kodiert.

6. Isolierte Nucleinsäure, bestehend aus einer Nucleotid-Sequenz von SEQ ID Nr: 59, welche für das Fusionsprotein Thio/HTLV-I gp21, wie in Anspruch 2 spezifiziert, kodiert.

7. Isolierte Nucleinsäure, bestehend aus einer Nucleotid-Sequenz von SEQ ID Nr: 58, welche für das Fusionsprotein GST/HTLV-II gp21, wie in Anspruch 3 spezifiziert, kodiert.

8. Isolierte Nucleinsäure, bestehend aus einer Nucleotid-Sequenz von SEQ ID Nr: 60, welche für das Fusionsprotein Thio/HTLV-II gp21, wie in Anspruch 4 spezifiziert; kodiert.

9. Expressionsvektor, umfassend eine Nucleinsäure, welche für das Fusionsprotein GST/HTLV-I gp21, wie in Anspruch 1 spezifiziert, kodiert, funktionsfähig gebunden an ein Nucleotidsequenz-Regulatorelement, welches die Expression der Nucleinsäure steuert, worin die Nucleinsäure die Nucleotid-Sequenz von SEQ ID Nr: 57 umfasst.

10. Expressionsvektor nach Anspruch 9, worin der Expressionsvektor in der American Type Culture Collection hinterlegt ist und als PTA-5238 bezeichnet ist.

11. Expressionsvektor, umfassend eine Nucleinsäure, welche für das Fusionsprotein Thio/HTLV-I gp21, wie in Anspruch 2 spezifiziert, kodiert, funktionsfähig gebunden an ein Nucleotidsequenz-Regulatorelement, welches die Expression der Nucleinsäure steuert, worin die Nucleinsäure die Nucleotid-Sequenz von SEQ ID Nr: 59 umfasst.

12. Expressionsvektor nach Anspruch 11, worin der Expressionsvektor in der American Type Culture Collection hinterlegt ist und als PTA-5240 bezeichnet ist.

13. Expressionsvektor, umfassend eine Nucleinsäure, welche für das Fusionsprotein GST/HTLV-II gp21, wie in Anspruch 3 spezifiziert, kodiert, funktionsfähig gebunden an ein Nucleotidsequenz-Regulatorelement, welches die Expression der Nucleinsäure steuert, worin die Nucleinsäure die Nucleotid-Sequenz von SEQ ID Nr: 58 umfasst.

14. Expressionsvektor nach Anspruch 13, worin der Expressionsvektor in der American Type Culture Collection hinterlegt ist und als PTA-5239 bezeichnet ist.

15. Expressionsvektor, umfassend eine Nucleinsäure, welche für das Fusionsprotein Thio/HTLV-II gp21. wie in Anspruch 4 spezifiziert, kodiert, funktionsfähig gebunden an ein Nucleotidsequenz-Regulatorelement, welches die Expression der Nucleinsäure steuert, worin die Nucleinsäure die Nucleotid-Sequenz von SEQ ID Nr: 60 umfasst.

16. Expressionsvektor nach Anspruch 15, worin der Expressionsvektor in der American Type Culture Collection hinterlegt ist und als PTA-5241 bezeichnet ist.

17. Verfahren zur Herstellung eines antigenen Fragments von HTLV gp21, umfassend:
Einführen eines Expressionsvektors, wie in einem der Ansprüche 9 bis 16 beansprucht, in eine Zelle;
Kultivieren der Zelle unter für die Herstellung eines Proteins geeigneten Bedingungen; und
Gewinnen des Proteins aus der Zellkultur unter Erhalt des antigenen Fragments von HTLV gp21.

18. Kit für die Detektierung von humanem T-lymphotropem Virus (HTLV), umfassend:
ein festes Substrat,
ein erstes HTLV gp21-Antigen-Fragment, immobilisiert auf dem festen Substrat,
eine Blocklösung zum Blocken der ungebundenen Region eines HTLV gp21-Antigen-Fragments auf dem festen Substrat;
ein zweites HTLV gp21-Antigen-Fragment;
eine Waschlösung, und
ein signalbildendes Mittel, funktionsfähig verbunden mit dem zweiten HTLV gp21-Antigen-Fragment zur Bildung eines Signals, worin die ersten und die zweiten HTLV gp21-Antigen-Fragmente ausgewählt sind aus Peptiden, wie in den Ansprüchen 1 bis 4 beansprucht.

19. Kit nach Anspruch 18, worin das erste HTLV gp21-Antigen-Fragment das Fusionsprotein Thio/HTLV-II gp21, wie in Anspruch 4 spezifiziert, ist und das zweite HTLV gp21-Antigen-Fragment das Fusionsprotein GST/ HTLV-I gp21, wie in Anspruch 1 spezifiziert ist, darstellt.

## Revendications

1. Peptide isolée constituée par une protéine fusionnée de glutathione S-transférase (GST) et un fragment antigénique de HTLV-1 gp21 avec la séquence amino acide SEQ ID n°5.

2. Peptide isolée constituée par une protéine fusionnée de thiorédoxine et un fragment antigénique de HTLV-1 gp21 avec la séquence aminoacide SEQ ID n°7.

3. Peptide isolée constituée par une protéine fusionnée de glutathione S tranférase et un fragment antigénique de HTLV-II gp21 avec une séquence amino acide SEQ ID n°6.

4. Peptide isolée constituée par une protéine fusionnée de thiorédoxine et un fragment antigène de HTLV-II gp21 avec une séquence amino acide SEQ ID n°8.

5. Acide nucléique isolé, constitué par une séquence nucléotide SEQ ID n°57 codant la protéine fusionnée GST/HTLV-1 gp21 selon la revendication 1.

6. Acide nucléique isolé, constitué par une séquence nucléotide SEQ ID n°59 codant la protéine fusionnée Thio/HTLV-1 gp21 selon la revendication 2.

7. Acide nucléique isolé, constitué par une séquence nucléotide SEQ ID n°58 codant la protéine fusionnée GST/HTLV-II gp21 selon la revendication 3.

8. Acide nucléique isolé, constitué par une séquence nucléotide SEQ ID n°60 codant la protéine fusionnée Thio/HTLV-II gp21 selon la revendication 4.

9. Vecteur d'expression, comprenant un acide nucléique codant la protéine fusionnée GST/HTLV-1 gp21 selon la revendication 1 lié fonctionnellement à un élément de régulation de séquence nucléotide qui contrôle l'expression de l'acide nucléique, l'acide nucléique comprenant la séquence nucléotide SEQ ID n°57.

10. Vecteur d'expression selon la revendication 9, dans lequel le vecteur d'expression est déposé dans la collection « American Type Culture Collection » sous la référence PTA-5238.

11. Vecteur d'expression, comprenant un acide nucléique codant la protéine fusionnée Thio/HTLV-1 gp21 selon la revendication 2 lié fonctionnellement à un élément de régulation de séquence nucléotide qui contrôle l'expression de l'acide nucléique, l'acide nucléique comprenant la séquence nucléotide SEQ ID n°59.

12. Vecteur d'expression selon la revendication 11, dans lequel le vecteur d'expression est déposé dans la collection « American Type Culture Collection » sous la référence PTA-5240.

13. Vecteur d'expression, comprenant un acide nucléique codant la protéine fusionnée GST/HTLV-II gp21 selon la revendication 3 lié fonctionnellement à un élément de régulation de séquence nucléotide qui contrôle l'expression de l'acide nucléique, l'acide nucléique comprenant la séquence nucléotide SEQ ID n°58.

14. Vecteur d'expression selon la revendication 13, dans lequel le vecteur d'expression est déposé dans la collection « American Type Culture Collection » sous la référence PTA-5239.

15. Vecteur d'expression, comprenant un acide nucléique codant la protéine fusionnée Thio/HTLV-II gp21 selon la revendication 4 lié fonctionnellement à un élément de régulation de séquence nucléotide qui contrôle l'expression de l'acide nucléique, l'acide nucléique comprenant la séquence nucléotide SEQ ID n°60.

16. Vecteur d'expression selon la revendication 15, dans lequel le vecteur d'expression est déposé dans la collection « American Type Culture Collection » sous la référence PTA-5241.

17. Procédé pour préparer un fragment antigénique de HTLV gp21 comprenant :
- l'introduction d'un vecteur d'expression selon l'une quelconque des revendications 9 à 16 dans une cellule ;
- la culture de la cellule dans des conditions appropriées pour produire une protéine ; et
- la récupération de la protéine de la culture de la cellule pour obtenir le fragment d'antigène de HTLV gp21.

18. Kit pour la détection du virus humain T-lymphotropique (HTLV) comprenant :
- un substrat solide,
- un premier fragment antigénique de HTLV gp21 immobilisé sur le substrat solide,
- une solution de blocage pour bloquer une région non liée du fragment antigénique de HTLV gp21 sur le substrat solide,
- un second fragment antigénique de HTLV gp21,
- une solution de lavage, et
- des moyens de production de signal liés fonctionnellement au second fragment antigénique de HTLV gp21 pour produire un signal, dans lequel le premier et le second fragment antigénique de HTLV gp 21 sont choisis parmi les peptides selon les revendications 1 à 4.

19. Kit selon la revendication 18, dans lequel le premier fragment antigénique de HTLV gp21 est la protéine fusionnée Thio/HTLV-II gp21 selon la revendication 4 et le second fragment antigénique de HTLV gp 21 est la protéine fusionnée GST/HTLV-1 gp21 selon la revendication 1.


### Sequence Listing

<110>Development center for biotechnology
   <120>Fusion protein of human T-lymthotropic virus
<160>62
<210>1
   <211>164
   <212>PRT
   <213>Human T-lymphotrophic virus type I
<220>
   <221>PEPTIDE
<300>
   <301>Malik,K.T., Even,J. and Karpas,A.
   <302>Molecular cloning and complete nucleotide sequence of an adult T cell leukaemia virus/human T cell leukaemia virus type I (ATLV/HTLV-I) isolate of Caribbean origin: relationship to other members of the ATLV/HTLV-I subgroup
   <303> J. Gen. Virol.
   <304>69
   <305>Pt 7
   <307>1988
   <308>D13784
<400>1
<210>2
   <211>166
   <212>PRT
   <213>Human T-lymphotrophic virus type II
<220>
   <221>PEPTIDE
<300>
   <301>Shimotohno,K., Golde, D.W., Miwa,M., Sugimura,T. and Chen, I.S.
   <302>Nucleotide sequence analysis of the long terminal repeat of human T-cell leukemia virus type II
   <303> Proc. Natl. Acad. Sci. U.S.A
   <304>81
   <305>4
   <306>1079-1083
   <307>1984
   <308>NC_001488
<400>2
<210>3
   <211>89
   <212>PRT
   <213>Human T-lymphotrophic virus type I
<220>
   <221>DOMAIN
<400>3
   Ala Ser Gly Lys Ser Leu Leu His Glu Val 10
   Asp Lys Asp Ile Ser Gln Leu Thr Gln Ala 20
   Ile Val Lys Asn His Lys Asn Leu Leu Lys 30
   Ile Ala Gln Tyr Ala Ala Gln Asn Arg Arg 40
   Gly Leu Asp Leu Leu Phe Trp Glu Gln Gly 50
   Gly Leu Cys Lys Ala Leu Gln Glu Gln Cys 60
   Cys Phe Leu Asn Ile Thr Asn Ser His Val 70
   Ser Ile Leu Gln Glu Arg Pro Pro Leu Glu 80
   Asn Arg Val Leu Thr Gly Trp Gly Leu     89
<210>4
   <211>89
   <212>PRT
   <213> Human T-lymphotrophic virus type II
<220>
   <221>DOMAIN
<400>4
   Ala Ser Ser Lys Ser Leu Leu Phe Glu Val 10
   Asp Lys Asp Ile Ser His Leu Thr Gln Ala 20
   Ile Val Lys Asn His Gln Asn Ile Leu Arg 30
   Val Ala Gln Tyr Ala Ala Gln Asn Arg Arg 40
   Gly Leu Asp Leu Leu Phe Trp Glu Gln Gly 50
   Gly Leu Cys Lys Ala Ile Gln Glu Gln Cys 60
   Cys Phe Leu Asn Ile Ser Asn Thr His Val 70
   Ser Val Leu Gln Glu Arg Pro Pro Leu Glu 80
   Lys Arg Val Ile Thr Gly Trp Gly Leu     89
<210>5
   <211>336
   <212>PRT
   <213>artificial sequence
<220>
   <221>CHAIN
   <223>Glutahion S-transferase/ Human T-lymphotrophic virus type I gp21 fusion protein
<400>5
   Met Ser Pro Ile Leu Gly Tyr Trp Lys Ile
   1 5 10
   Lys Gly Leu Val Gln Pro Thr Arg Leu Leu
   15 20
   Leu Glu Tyr Leu Glu Glu Lys Tyr Glu Glu
   25 30
   His Leu Tyr Glu Arg Asp Glu Gly Asp Lys
   35 40
   Trp Arg Asn Lys Lys Phe Glu Leu Gly Leu
   45 50
   Glu Phe Pro Asn Leu Pro Tyr Tyr Ile Asp
   55 60
   Gly Asp Val Lys Leu Thr Gln Ser Met Ala
   65 70
   Ile Ile Arg Tyr Ile Ala Asp Lys His Asn
   75 80
   Met Leu Gly Gly Cys Pro Lys Glu Arg Ala
   85 90
   Glu Ile Ser Met Leu Glu Gly Ala Val Leu
   95 100
   Asp Ile Arg Tyr Gly Val Ser Arg Ile Ala
   105 110
   Tyr Ser Lys Asp Phe Glu Thr Leu Lys Val
   115 120
   Asp Phe Leu Ser Lys Leu Pro Glu Met Leu
   125 130
   Lys Met Phe Glu Asp Arg Leu Cys His Lys
   135 140
   Thr Tyr Leu Asn Gly Asp His Val Thr His
   145 150
   Pro Asp Phe Met Leu Tyr Asp Ala Leu Asp
   155 160
   Val Val Leu Tyr Met Asp Pro Met Cys Leu
   165 170
   Asp Ala Phe Pro Lys Leu Val Cys Phe Lys
   175 180
   Lys Arg Ile Glu Ala Ile Pro Gln Ile Asp
   185 190
   Lys Tyr Leu Lys Ser Ser Lys Tyr Ile Ala
   195 200
   Trp Pro Leu Gln Gly Trp Gln Ala Thr Phe
   205 210
   Gly Gly Gly Asp His Pro Pro Lys Ser Asp
   215 220
   Leu Val Pro Arg Gly Ser Pro Gly Ile Ser
   225 230
   Gly Gly Gly Gly Gly Ile Leu Asp Ser Met
   235 240
   Gly Ala Ser Gly Lys Ser Leu Leu His Glu
   245 250
   Val Asp Lys Asp Ile Ser Gln Leu Thr Gln
   255 260
   Ala Ile Val Lys Asn His Lys Asn Leu Leu
   265 270
   Lys Ile Ala Gln Tyr Ala Ala Gln Asn Arg
   275 280
   Arg Gly Leu Asp Leu Leu Phe Trp Glu Gln
   285 290
   Gly Gly Leu Cys Lys Ala Leu Gln Glu Gln
   295 300
   Cys Cys Phe Leu Asn Ile Thr Asn Ser His
   305 310
   Val Ser Ile Leu Gln Glu Arg Pro Pro Leu
   315 320
   Glu Asn Arg Val Leu Thr Gly Trp Gly Leu
   325 330
   Lys Leu Asn Ser Ser Stop
   335
<210>6
   <211>321
   <212>PRT
   <213>artificial sequence
<220>
   <221>CHAIN
   <223>Glutathione S-transferase/ Human T-lymphotrophic virus type II gp 21 fusion protein
<400>6 Stop
<210>7
   <211>253
   <212>PRT
   <213>artificial sequence
<220>
   <221>CHAIN
   <223>Thioredoxin/Human T-lymphotrophic virus type I gp21 fusion protein
<400>7
   Met Ser Asp Lys Ile Ile His Leu Thr Asp
   1 5 10
   Asp Ser Phe Asp Thr Asp Val Leu Lys Ala
   15 20
   Asp Gly Ala Ile Leu Val Asp Phe Trp Ala
   25 30
   His Trp Cys Gly Pro Cys Lys Met Ile Ala
   35 40
   Pro Ile Leu Asp Glu Ile Ala Asp Glu Tyr
   45 50
   Gln Gly Lys Leu Thr Val Ala Lys Leu Asn
   55 60
   Ile Asp His Asn Pro Gly Thr Ala Pro Lys
   65 70
   Tyr Gly Ile Arg Gly Ile Pro Thr Leu Leu
   75 80
   Leu Phe Lys Asn Gly Glu Val Ala Ala Thr
   85 90
   Lys Val Gly Ala Leu Ser Lys Gly Gln Leu
   95 100
   Lys Glu Phe Leu Asp Ala Asn Leu Ala Gly
   105 110
   Ser Gly Ser Gly Asp Asp Asp Asp Lys Val
   115 120
   Pro Met Gly Ser Ser His His His His His
   125 130
   His Ser Ser Gly Leu Val Pro Arg Gly Ser
   135 140
   His Met Ala Ser Gly Lys Ser Leu Leu His
   145 150
   Glu Val Asp Lys Asp Ile Ser Gln Leu Thr
   155 160
   Gln Ala Ile Val Lys Asn His Lys Asn Leu
   165 170
   Leu Lys Ile Ala Gln Tyr Ala Ala Gln Asn
   175 180
   Arg Arg Gly Leu Asp Leu Leu Phe Trp Glu
   185 190
   Gln Gly Gly Leu Cys Lys Ala Leu Gln Glu
   195 200
   Gln Cys Cys Phe Leu Asn Ile Thr Asn Ser
   205 210
   His Val Ser Ile Leu Gln Glu Arg Pro Pro
   215 220
   Leu Glu Asn Arg Val Leu Thr Gly Trp Gly
   225 230
   Leu Leu Glu Ile Phe Glu Phe Arg Gly Arg
   235 240
   Arg Pro Leu Glu Ser Thr Cys Ser Asn Arg
   245 250
   Thr Gly Stop
<210>8
   <211>253
   <212>PRT
   <213>artificial sequence
<220>
   <221>CHAIN
   <223>Thioredoxin/Human T-lymphotrophic virus type II gp21 fusion protein
<400>8
   Met Ser Asp Lys Ile Ile His Leu Thr Asp
   1 5 10
   Asp Ser Phe Asp Thr Asp Val Leu Lys Ala
   15 20
   Asp Gly Ala Ile Leu Val Asp Phe Trp Ala
   25 30
   His Trp Cys Gly Pro Cys Lys Met Ile Ala
   35 40
   Pro Ile Leu Asp Glu Ile Ala Asp Glu Tyr
   45 50
   Gln Gly Lys Leu Thr Val Ala Lys Leu Asn
   55 60
   Ile Asp His Asn Pro Gly Thr Ala Pro Lys
   65 70
   Tyr Gly Ile Arg Gly Ile Pro Thr Leu Leu
   75 80
   Leu Phe Lys Asn Gly Glu Val Ala Ala Thr
   85 90
   Lys Val Gly Ala Leu Ser Lys Gly Gln Leu
   95 100
   Lys Glu Phe Leu Asp Ala Asn Leu Ala Gly
   105 110
   Ser Gly Ser Gly Asp Asp Asp Asp Lys Val
   115 120
   Pro Met Gly Ser Ser His His His His His
   125 130
   His Ser Ser Gly Leu Val Pro Arg Gly Ser
   135 140
   His Met Ala Ser Ser Lys Ser Leu Leu Phe
   145 150
   Glu Val Asp Lys Asp Ile Ser His Leu Thr
   155 160
   Gln Ala Ile Val Lys Asn His Gln Asn Ile
   165 170
   Leu Arg Val Ala Gln Tyr Ala Ala Gln Asn
   175 180
   Arg Arg Gly Leu Asp Leu Leu Phe Trp Glu
   185 190
   Gln Gly Gly Leu Cys Lys Ala Ile Gln Glu
   195 200
   Gln Cys Cys Phe Leu Asn Ile Ser Asn Thr
   205 210
   His Val Ser Val Leu Gln Glu Arg Pro Pro
   215 220
   Leu Glu Lys Arg Val Ile Thr Gly Trp Gly
   225 230
   Leu Leu Glu Ile Phe Glu Phe Arg Gly Arg
   235 240
   Arg Pro Leu Glu Ser Thr Cys Ser Asn Arg
   245 250
   Thr Gly Stop
<210>9
   <211>492
   <212>DNA
   <213>artificial sequence
<220>
   <221>CDS
   <223>antigenic fragment of Human T-lymphotrophic virus type Igp21 modified with E. coli preferred codons
   <400>9
   atgggtgcag gcgttgctgg cggtatcacc ggctccatgt ccctggcatc 50
<210>10
   <211>40
   <212>DNA
   <213> artificial sequence
<220>
   <221>primer_bind
   <223>I F1
   <400>10
   cgcatatggg tgcaggcgtt gctggcggta tcaccggctc 40
<210>11
   <211>40
   <212>DNA
   <213>artificial sequence
<220>
   <221>primer_bind
   <223>I F2
<400>11
   tggcatccgg taaatctctg ctgcacgaag ttgacaaaga 40
<210>12
   <211>41
   <212>DNA
   <213>artificial sequence
<220>
   <221>primer_bind
   <223>I F3
<400>12
   agctgactca ggcaatcgtt aaaaaccaca aaaacctgc t 41
<210>13
   <211>40
   <212>DNA
   <213>artificial sequence
<220>
   <221>primer_bind
   <223>I F4
<400>13
   cgcagtacgc tgcacagaac cgtcgtggcc tggacctgct 40
<210>14
   <211>40
   <212>DNA
   <213>artificial sequence
<220>
   <221>primer_bind
   <223>I F5
<400>14
   aacagggtgg cctgtgcaaa gcactgcagg aacagtgctg 40
<210>15
   <211>40
   <212>DNA
   <213>artificial sequence
<220>
   <221>primer_bind
   <223>I F6
<400>15
   acatcactaa ctcccacgtt tctatcctgc aggaacgtcc 40
<210>16
   <211>40
   <212>DNA
   <213>artificial sequence
<220>
   <221>primer_bind
   <223>I F7
<400>16
   aaaaccgtgt actgactggc tggggcctga actgggacct 40
<210>17
   <211>40
   <212>DNA
   <213>artificial sequence
<220>
   <221> primer_bind
   <223>I F8
<400>17
   ctcagtgggc tcgtgaggcg ctgcagactg gtatcaccct 40
<210>18
   <211>40
   <212>DNA
   <213>artificial sequence
<220>
   <221>primer_bind
   <223>I F9
<400>18
   tgctgctgct ggttatcctg gcaggtccgt gcatcctgcg 40
<210>19
   <211>40
   <212>DNA
   <213>artificial sequence
<220>
   <221>primer_bind
   <223>I F10
<400>19
   gtcacctgcc gtctcgtgta cgttacccgc actactctct 40
<210>20
   <211>49
   <212>DNA
   <213>artificial sequence
<220>
   <221>primer_bind
   <223>I R1
<400>20
   cgctcgagtt acagggaaga ttccggtttg atcagagagt agtgcgggt 49
<210>21
   <211>40
   <212>DNA
   <213>artificial sequence
<220>
   <221>primer_bind
   <223>I R2
<400>21
   cgagacggca ggtgacgcag ctgacgcagg atgcacggac 40
<210>22
   <211>40
   <212> DNA
   <213>artificial sequence
<220>
   <221>primer_bind
   <223>I R3
<400>22
   ataaccagca gcagcagcgc aaccagggtg ataccagtct 40
<210>23
   <211>40
   <212> DNA
   <213>artificial sequence
<220>
   <221>primer_bind
   <223>I R4
<400>23
   tcacgagccc actgagacag gcccaggtcc cagttcaggc 40
<210>24
   <211>40
   <212>DNA
   <213>artificial sequence
<220>
   <221> primer_bind
   <223>I R5
<400>24
   gtcagtacac ggttttccag cggcggacgtt cctgcagga 40
<210>25
   <211>40
   <212>DNA
   <213>artificial sequence
<220>
   <221>primer_bind
   <223 >I R6
<400>25
   tgggagttag tgatgttcag gaaacagcac tgttcctgca 40
<210>26
   <211>40
   <212>DNA
   <213>artificial sequence
<220>
   <221>primer_bind
   <223>I R7
<400>26
   cacaggccac cctgttccca gaacagcagg tccaggccac 40
<210>27
   <211>40
   <212>DNA
   <213>artificial sequence
<220>
   <221>pimer_bind
   <223>I R8
<400>27
   tgtgcagcgt actgcgcgat tttcagcagg tttttgtggt 40
<210>28
   <211>40
   <212>DNA
   <213>artificial sequence
<220>
   <221>primer_bind
   <223>I R9
<400>28
   attgcctgag tcagctggga gatgtctttg tcaacttcgt 40
<210>29
   <211>40
   <212>DNA
   <213>artificial sequence
<220>
   <221>primer_bind
   <223>1 R10
<400>29
   gatttaccgg atgccaggga catggagccg gtgataccgc 40
<210>30
   <211>501
   <212>DNA
   <213>artificial sequence
<220>
   <221>CDS
   <223> Human T-lymphotrophic virus type II gp21
<400>30
   atggccggga caggtatcgc tggcggagta acaggctccc tatctctagc 50
   ttccagtaaa agccttctct tcgaggttga caaagatatc tcccacctta 100
   cccaggccat agtcaaaaat catcaaaaca tcctccgggt tgcacaatat 150
   gcagcccaga atagacgagg attagacctc ctattctggg aacaaggggg 200
   tttgtgcaaa gccatacagg agcaatgttg cttcctcaat atcagtaaca 250
   ctcatgtatc cgtcctccaa gaacggcccc ctcttgaaaa gcgtgtcatc 300
   accggttggg gactaaactg ggatcttggt ctgtcccagt gggcacgaga 350
   agccctccag acaggcataa ccattctcac cctactcctc cttgtcatat 400
   tgtttggccc ctgcatcctc cgccaaatcc aagcccttcc gcagcggtta 450
   caaaaccgac atagccagta tgcccttatc aaccaagaga ccatgctata 500
   a                                                       501
<210>31
   <211>40
   <212>DNA
   <213>artificial sequence
<220>
   <221>primer_bind
   <223>II F1
<400>31
   cgcatatggc cgggacaggt atcgctggcg gagtaacagg 40
<210>32
   <211>40
   <212>DNA
   <213>artificial sequence
<220>
   <221>primer_bind
   <223 >II F2
<400>32
   ctagcttcca gtaaaagcct tctcttcgag gttgacaaag 40
<210>33
   <211>40
   <212>DNA
   <213>artificial sequence
<220>
   <221>prmer_bind
   <223>II F3
<400>33
   ccttacccag gccatagtca aaaatcatca aaacatcctc 40
<210>34
   <211>40
   <212>DNA
   <213>artificial sequence
<220>
   <221>primer_bind
   <223>II F 4
<400>34
   aatatgcagc ccagaataga cgaggattag acctcctatt 40
<210>35
   <211>40
   <212>DNA
   <213>artificial sequence
<220>
   <221>primer_bind
   <223>II F5
<400>35
   gggggtttgt gcaaagccat acaggagcaa tgttgcttcc 40
<210>36
   <211>40
   <212>DNA
   <213>artificial sequence
<220>
   <221>primer_bind
   <223>II F6
<400>36
   taacactcat gtatccgtcc tccaagaacg gccccctctt 40
<210>37
   <211>40
   <212>DNA
   <213>artificial sequence
<220>
   <221>primer_bind
   <223>II F7
<400>37
   tcatcaccgg ttggggacta aactgggatc ttggtctgtc 40
<210>38
   <211>40
   <212>DNA
   <213>artificial sequence
<220>
   <221>primer_bind
   <223>II F8
<400>38
   cgagaagccc tccagacagg cataaccatt ctcaccctac 40
<210>39
   <211>40
   <212>DNA
   <213>artificial sequence
<220>
   <221>primer_bind
   <223>II F9
<400>39
   catattgttt ggcccctgca tcctccgcca aatccaagcc 40
<210>40
   <211>40
   <212>DNA
   <213>artificial sequence
<220>
   <221>primer_bind
   <223>II F10
<400>40
   ggttacaaaa ccgacatagc cagtatgccc ttatcaacca 40
<210>41
   <211>39
   <212>DNA
   <213>artificial sequence
<220>
   <221>primer_bind
   <223>II R1
<400>41
   cgctcgagtt atagcatggt ctcttggttg ataagggca 39
<210>42
   <211>40
   <212>DNA
   <213>artificial sequence
<220>
   <221>primer_bind
   <223>II R2
<400>42
   gtcggttttg taaccgctgc ggaagggctt ggatttggcg 40
<210>43
   <211>40
   <212>DNA
   <213>artificial sequence
<220>
   <221>primer_bind
<400>43
   gggccaaaca atatgacaag gaggagtagg gtgagaatgg 40
<210>44
   <211>40
   <212>DNA
   <213>artificial sequence
<220>
   <221>primer_bind
   <223>II R4
<400>44
   ctggagggct tctcgtgccc actgggacag accaagatcc 40
<210>45
   <211>40
   <212>DNA
   <213>artificial sequence
<220>
   <221>primer_bind
   <223>II R5
<400>45
   cccaaccggt gatgacacgc ttttcaagag ggggccgttc 40
<210>46
   <211>40
   <212>DNA
   <213>artificial sequence
<220>
   <221>primer_bind
   <223>II R6
<400>46
   gatacatgag tgttactgat attgaggaag caacattgct 40
<210>47
   <211>40
   <212>DNA
   <213>artificial sequence
<220>
   <221>primer_bind
   <223>II R7
<400>47
   tttgcacaaa cccccttgtt cccagaatag gaggtctaat 40
<210>48
   <211>40
   <212>DNA
   <213>artificial sequence
<220>
   <221>primer_bind
   <223>II R8
<400>48
   tctgggctgc atattgtgca acccggagga tgttttgatg 40
<210>49
   <211>40
   <212>DNA
   <213>artificial sequence
<220>
   <221>primer_bind
   <223>II R9
<400>49
   atggcctggg taaggtggga gatatctttg tcaacctcga 40
<210>50
   <211>40
   <212>DNA
   <213>artificial sequence
<220>
   <221> primer_bind
   <223>II R10
<400>50
   tttactggaa gctagagata gggagcctgt tactccgcca 40
<210>51
   <211>31
   <212>DNA
   <213>artificial sequence
<220>
   <221>primer_bind
   <223>I gp21 (270)/GST F
<400>51
   cgccatgggt gcatccggta aatctctgct g 31
<210>52
   <211>29
   <212>DNA
   <213>artificial sequence
<220>
   <221>primer_bind
   <223>I gp21 (270)/GST R
<400>52
   cgaagcttca ggccccagcc agtcagtac 29
<210>53
   <211>29
   <212>DNA
   <213>artificial sequence
<220>
   <221>primer_bind
   <223>II gp21 (270)/GST F
<400>53
   cgggatccgc ttccagtaaa agccttctc 29
<210>54
   <211>29
   <212>DNA
   <213>artificial sequence
<220>
   <221>primer_bind
   <223>II gp21 (270)/GST R
<400>54
   cgaagcttta gtccccaacc ggtgatgac 29
<210>55
   <211>267
   <212> DNA
   <213> artificial sequence
<220>
   <221>Misc_feature
   <223>sequence encoding an antigenic fragment of Human T-lymphotrophic virus type II gp21
<400>55
   gcatccggta aatctctgct gcacgaagtt gacaaagaca tctcccagct 50
   gactcaggca atcgttaaaa accacaaaaa cctgctgaaa atcgcgcagt 100
   acgctgcaca gaaccgtcgt ggcctggacc tgctgttctg ggaacagggt 150
   ggcctgtgca aagcactgca ggaacagtgc tgtttcctga acatcactaa 200
   ctcccacgtt tctatcctgc aggaacgtcc gccgctggaa aaccgtgtac 250
   tgactggctg gggcctg 267
<210>56
   <211>267
   <212>DNA
   <213>artificial sequence
<220>
   <221> Misc_feature
   <223> sequence encoding an antigenic fragment of Human T-lymphotrophic virus type II gp21
<400>56
   gcttccagta aaagccttct cttcgaggtt gacaaagata tctcccacct 50
   tacccaggcc atagtcaaaa atcatcaaaa catcctccgg gttgcacaat 100
   atgcagccca gaatagacga ggattagacc tcctattctg ggaacaaggg 150
   ggtttgtgca aagccataca ggagcaatgt tgcttcctca atatcagtaa 200
   cactcatgta tccgtcctcc aagaacggcc ccctcttgaa aagcgtgtca 250
   tcaccggttg gggacta                                    267
<210>57
   <211>1008
   <212>DNA
   <213>artificial sequence
<220>
   <221> CDS
   <223>sequence endocing GST/HTLV-I gp21 fusion protein
<400>57
   atgtccccta tactaggtta ttggaaaatt aagggccttg tgcaacccac 50
   tcgacttctt ttggaatatc ttgaagaaaa atatgaagag catttgtatg 100
   agcgcgatga aggtgataaa tggcgaaaca aaaagtttga attgggtttg 150
   gagtttccca atcttcctta ttatattgat ggtgatgtta aattaacaca 200
   gtctatggcc atcatacgtt atatagctga caagcacaac atgttgggtg 250
   gttgtccaaa agagcgtgca gagatttcaa tgcttgaagg agcggttttg 300
   gatattagat acggtgtttc gagaattgca tatagtaaag actttgaaac 350
   tctcaaagtt gattttctta gcaagctacc tgaaatgctg aaaatgttcg 400
   aagatcgttt atgtcataaa acatatttaa atggtgatca tgtaacccat 450
   cctgacttca tgttgtatga cgctcttgat gttgttttat acatggaccc 500
   aatgtgcctg gatgcgttcc caaaattagt ttgttttaaa aaacgtattg 550
   aagctatccc acaaattgat aagtacttga aatccagcaa gtatatagca 600
   tggcctttgc agggctggca agccacgttt ggtggtggcg accatcctcc 650
   aaaatcggat ctggttccgc gtggatcccc gggaatttcc ggtggtggtg 700
   gtggaattct agactccatg ggtgcatccg gtaaatctct gctgcacgaa 750
   gttgacaaag acatctccca gctgactcag gcaatcgtta aaaaccacaa 800
   aaacctgctg aaaatcgcgc agtacgctgc acagaaccgt cgtggcctgg 850
   acctgctgtt ctgggaacag ggtggcctgt gcaaagcact gcaggaacag 900
   tgctgtttcc tgaacatcac taactcccac gtttctatcc tgcaggaacg 950
   tccgccgctg gaaaaccgtg tactgactgg ctggggcctg aagcttaatt 1000
   catcgtga 1008
<210>58
   <211>963
   <212>DNA
   <213>artificial sequence
<220>
   <221>CDS
   <223> sequence encoding GST/HTLV-II gp21 fusion protein
<400>58
   atgtccccta tactaggtta ttggaaaatt aagggccttg tgcaacccac 50
   tcgacttctt ttggaatatc ttgaagaaaa atatgaagag catttgtatg 100
   agcgcgatga aggtgataaa tggcgaaaca aaaagtttga attgggtttg 150
   gagtttccca atcttcctta ttatattgat ggtgatgtta aattaacaca 200
   gtctatggcc atcatacgtt atatagctga caagcacaac atgttgggtg 250
   gttgtccaaa agagcgtgca gagatttcaa tgcttgaagg agcggttttg 300
   gatattagat acggtgtttc gagaattgca tatagtaaag actttgaaac 350
   tctcaaagtt gattttctta gcaagctacc tgaaatgctg aaaatgttcg 400
   aagatcgttt atgtcataaa acatatttaa atggtgatca tgtaacccat 450
   cctgacttca tgttgtatga cgctcttgat gttgttttat acatggaccc 500
   aatgtgcctg gatgcgttcc caaaattagt ttgttttaaa aaacgtattg 550
   aagctatccc acaaattgat aagtacttga aatccagcaa gtatatagca 600
   tggcctttgc agggctggca agccacgttt ggtggtggcg accatcctcc 650
   aaaatcggat ctggttccgc gtggatccgc ttccagtaaa agccttctct 700
   tcgaggttga caaagatatc tcccacctta cccaggccat agtcaaaaat 750
   catcaaaaca tcctccgggt tgcacaatat gcagcccaga atagacgagg 800
   attagacctc ctattctggg aacaaggggg tttgtgcaaa gccatacagg 850
   agcaatgttg cttcctcaat atcagtaaca ctcatgtatc cgtcctccaa 900
   gaacggcccc ctcttgaaaa gcgtgtcatc accggttggg gactaaagct 950
   taattcatcg tga 963
<210>59
   <211>759
   <212>DNA
   <213>artificial sequence
<220>
   <221>CDS
   <223>sequence encoding Thio/HTLV-I gp21 fusion protein
<400>59
   atgtctgata aaattattca tctgactgat gattcttttg atactgatgt 50
   acttaaggca gatggtgcaa tcctggttga tttctgggca cactggtgcg 100
   gtccgtgcaa aatgatcgct ccgattctgg atgaaatcgc tgacgaatat 150
   cagggcaaac tgaccgttgc aaaactgaac atcgatcaca acccgggcac 200
   tgcgccgaaa tatggcatcc gtggtatccc gactctgctg ctgttcaaaa 250
   acggtgaagt ggcggcaacc aaagtgggtg cactgtctaa aggtcagttg 300
   aaagagttcc tcgacgctaa cctggccggc tctggatccg gtgatgacga 350
   tgacaaggta cccatgggca gcagccatca tcatcatcat cacagcagcg 400
   gcctggtgcc gcgcggcagc catatggcat ccggtaaatc tctgctgcac 450
   gaagttgaca aagacatctc ccagctgact caggcaatcg ttaaaaacca 500
   caaaaacctg ctgaaaatcg cgcagtacgc tgcacagaac cgtcgtggcc 550
   tggacctgct gttctgggaa cagggtggcc tgtgcaaagc actgcaggaa 600
   cagtgctgtt tcctgaacat cactaactcc cacgtttcta tcctgcagga 650
   acgtccgccg ctggaaaacc gtgtactgac tggctggggc ctgctcgaga 700
   tcttcgaatt ccgcggccgc aggcctctag agtcgacctg cagtaatcgt 750
   acagggtag                                              759
<210>60
   <211>759
   <212>DNA
   <213>artificial sequence
<220>
   <221>CDS
   <223>sequence encoding Thio/HTLV-II gp21 fusion protein
<400>60
   atgtctgata aaattattca tctgactgat gattcttttg atactgatgt 50
   acttaaggca gatggtgcaa tcctggttga tttctgggca cactggtgcg 100
   gtccgtgcaa aatgatcgct ccgattctgg atgaaatcgc tgacgaatat 150
   cagggcaaac tgaccgttgc aaaactgaac atcgatcaca acccgggcac 200
   tgcgccgaaa tatggcatcc gtggtatccc gactctgctg ctgttcaaaa 250
   acggtgaagt ggcggcaacc aaagtgggtg cactgtctaa aggtcagttg 300
   aaagagttcc tcgacgctaa cctggccggc tctggatccg gtgatgacga 350
   tgacaaggta cccatgggca gcagccatca tcatcatcat cacagcagcg 400
   gcctggtgcc gcgcggcagc catatggctt ccagtaaaag ccttctcttc 450
   gaggttgaca aagatatctc ccaccttacc caggccatag tcaaaaatca 500
   tcaaaacatc ctccgggttg cacaatatgc agcccagaat agacgaggat 550
   tagacctcct attctgggaa caagggggtt tgtgcaaagc catacaggag 600
   caatgttgct tcctcaatat cagtaacact catgtatccg tcctccaaga 650
   acggccccct cttgaaaagc gtgtcatcac cggttgggga ctactcgaga 700
   tcttcgaatt ccgcggccgc aggcctctag agtcgacctg cagtaatcgt 750
   acagggtag                                              759